# EUROPEAN PATENT SPECIFICATION

(11) **EP 3 582 280 B1**
(45) Date of publication and mention of the grant of the patent: **20.03.2024**
(21) Application number: 18177827.5
(22) Date of filing: 14.06.2018
(51) Int. Cl.: H10K 85/60, H10K 50/165

(54) **ORGANIC MATERIAL FOR AN ELECTRONIC OPTOELECTRONIC DEVICE AND ELECTRONIC DEVICE COMPRISING THE ORGANIC MATERIAL**
ORGANISCHES MATERIAL FÜR EINE ELEKTRONISCHE OPTOELEKTRONISCHE VORRICHTUNG UND ELEKTRONISCHE VORRICHTUNG MIT DEM ORGANISCHEN MATERIAL
MATÉRIAU ORGANIQUE POUR DISPOSITIF OPTOÉLECTRONIQUE ÉLECTRONIQUE ET DISPOSITIF ÉLECTRONIQUE COMPRENANT LE MATÉRIAU ORGANIQUE

(43) Date of publication of application: 18.12.2019
(73) Proprietor: Novaled GmbH, 01099 Dresden (DE)
(72) Inventor: Elena GALAN, 01099 Dresden (DE); Benjamin SCHULZE, 01099 Dresden (DE)
(74) Representative: Michalski Hüttermann & Partner Patentanwälte mbB

(56) References cited:
- EP-A1- 3 109 919
- CN-A- 106 243 091
- JP-A- 2005 243 266
- US-A1- 2007 161 793
- HAI-TAO FENG ET AL: "White-Light Emission of a Binary Light-Harvesting Platform Based on an Amphiphilic Organic Cage", CHEMISTRY OF MATERIALS, vol. 30, no. 4, 8 February 2018 (2018-02-08), pages 1285-1290, XP055524984, ISSN: 0897-4756, DOI: 10.1021/acs.chemmater.7b04703

## Description

### Technical Field

The present invention relates to an organic material and to an electronic device comprising the organic material, particularly to an electroluminescent device, particularly to an organic light emitting diode (OLED); the invention pertains also to a device comprising the electric device and/or the electroluminescent device, particularly to a display device, particularly to a display device comprising the OLED.

### Background Art

Organic light-emitting diodes (OLEDs), which are self-emitting devices, have a wide viewing angle, excellent contrast, quick response, high brightness, excellent driving voltage characteristics, and color reproduction. A typical OLED includes an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. There is continuing demand for development of improved materials, with the aim that operational voltage is as low as possible while brightness/luminance is high, and that injection and flow of holes and electrons is balanced, so that an OLED having the above-described structure has excellent efficiency and/or a long lifetime.

One of well-established approaches for achieving low operational voltages and high current densities/luminances is electrical p- and/or n-doping in charge injection/charge transport layers, and especially redox doping which generates doped layers with high charge carrier concentrations.

Compounds for electroluminescent devices are inter alia disclosed in JP 2005 243 266 A, CN 106 243 091 A, US 2007/161793 A1 and EP 3 109 919 A1.

JP 2005 243266 A discloses a compound according to 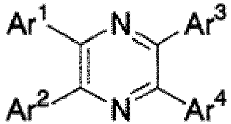 as the electron transport material, wherein Ar¹ to Ar⁴ denote aromatic hydrocarbon groups or aromatic heterocyclic group expressed bv 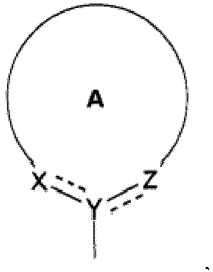 wherein the cycle A denotes an aromatic hydrocarbon group or an aromatic heterocyclic group which may have a substituent. X, Z denotes -CR¹=, -NR²-, -O-, or -S- , and Y denotes a carbon atom or a nitrogen atom, and denotes a single bond or a double bond independently from X and Z. EP 3 109 919 A1 discloses a semiconducting material comprising (i) in substantially elemental form, an electropositive element selected from alkaline metals, alkaline earth metals, rare earth metals, and transition metals, and (ii) at least one first compound which is a compound comprising at least one polar group selected from phosphine oxide group or diazole group; a process for manufacturing the semiconducting material; an electronic device comprising a cathode, an anode and the semiconducting material.

### DISCLOSURE

Aspects of the present invention provide a compound for an electronic device, particularly for a light-emitting or electroluminescent device comprising an emission layer and at least two electrodes, for increasing the efficiency, such as the external quantum efficiency EQE, and for achieving low operating voltage and long lifetime, particularly in top and/or bottom emission organic light-emitting diodes (OLEDs).

Another aspect of the present invention provides an electronic device, particularly an electroluminescent device comprising the inventive compound. Still another aspect of the present invention provides a display device comprising the electroluminescent device. According to an aspect of the present invention, there is provided a compound for an electroluminescent device comprising at least one structural moiety A and at least one structural moiety B, wherein A and B do not share a common atom,
whereby the structural moiety A is a structural moiety having generic formula (I) 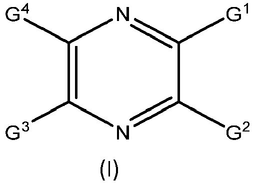
wherein G¹, G², G³ and G⁴ are independently selected from substituted or unsubstituted aryl comprising one to three aromatic rings, whereby one of the G¹, G², G³ and G⁴ is a phenylene unit and the others of the G¹, G², G³ and G⁴ are phenyl;
and wherein the structural moiety B is a structural moiety selected from substituted or unsubstituted phosphine oxide, phosphine sulfide, pyrimidine, pyridazine, naphthtalene, cinnoline, phtalazine, quinazoline, triazine, benzofurane, benzothiophene, dibenzofurane, dibenzothiophene, naphtofurane, naphtothiophene, naphtobenzofurane, naphtobenzothiophene, dinaphtofurane, dinaphtothiophene, C₆-C₆₀ aryl substituted with at least one group selected from pyridyl and nitrile , aryl consisting of 3, 4 or 5 condensed 6-membered aromatic rings and heteroaryl consisting of 3, 4 or 5 condensed 6-membered aromatic rings and comprising 1, 2 or 3 nitrogen ring atoms.

In the present specification, when a definition is not otherwise provided, "substituted" refers to one substituted with a deuterium, C₁ to C₁₂ alkyl and C₁ to C₁₂ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups,which themselves may be substituted with one or more aryl and/or heteroaryl groups.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group or a C₁ to C₆ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a t-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenylyl, and polycyclic groups comprising fused rings, like naphtyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common sp²-hybridized carbon atoms

In the present specification, the single bond refers to a direct bond.

In the context of the present invention, "different" means that the compounds do not have an identical chemical structure.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electron formed in the cathode may be easily injected into the emission layer and transported in the emission layer due to conductive characteristics according to a lowest unoccupied molecular orbital (LUMO) level.

### Advantageous Effects

Surprisingly, it was found that the organic material according to the invention solves the problem underlying the present invention by enabling devices in various aspects superior over the organic electroluminescent devices known in the art, in particular with respect to lifetime.

According to a further embodiment of the present invention, the compound comprises only one structural moiety A.

According to a further embodiment, the compound comprises one or two structural moieties B.

According to a further embodiment, three of the G¹, G², G³ and G⁴ are phenyl. It goes without saying that in case that there is more than one structural moiety A, then the representative substituents G¹ to G⁴ may be independent for any of the moieties A.

In an embodiment, one of G¹, G², G³ and G⁴ is a phenylene group and the others of G¹, G², G³ and G⁴ are phenyl groups.

According to a further embodiment, at least one of the moieties A and B are connected with each other via a single bond or a phenylene, biphenylene or terphenylene bridge.

According to a further embodiment, at least one of the G¹, G², G³ and G⁴ is substituted with at least one fluor, alkyl, fluoroalkyl, nitrile, subsituted or unsubstituted phosphine oxide and/or substituted or unsubstituted phosphine sulfide group.

According to a further embodiment, at least one of the moieties B is substituted with at least one fluor, alkyl, fluoroalkyl, nitrile, subsituted or unsubstituted phosphine oxide and/or substituted or unsubstituted phosphine sulfide group.

The inventors have surprisingly found that particularly good performance can be achieved when using the organic material according to the invention in an electron transport layer in an optoelectronic device.

Additionally or alternatively the inventors have surprisingly found that particularly good performance can be achieved when using the organic organic material according to the invention in or as an hole blocking layer in an optoelectronic device.

The present invention furthermore relates to an electronic device comprising a first electrode, a second electrode, and arranged between the first and second electrode, a layer comprising the organic material according to the invention.

According to a further embodiment, the electronic device comprises a hole blocking layer comprising a compound according to the invention.

According to a further embodiment, the electronic device comprises an electron transport layer comprising a compound according to the invention. According to a further embodiment, the electronic device is an electroluminescent device, preferably an organic light emitting diode.

The present invention furthermore relates to a display device comprising an electronic device according to the present invention, preferably, the display device comprises an organic light emitting diode according to the present invention.

The specific arrangements mentioned herein as preferred were found to be particularly advantageous.

Further an organic electroluminescent device having high efficiency and/or long life-span may be realized.

Hereinafter, the inventive compound and the device comprising it are described in more detail

### Compound

Similar as other compounds comprised in the inventive device outside the emitting layer, the compound of the present invention may not emit light under the operation condition of an electroluminescent device, for example an OLED.

Particularly preferred may be compounds with the following structures 1-1 to 1-35 and 1-39 to 1-48:

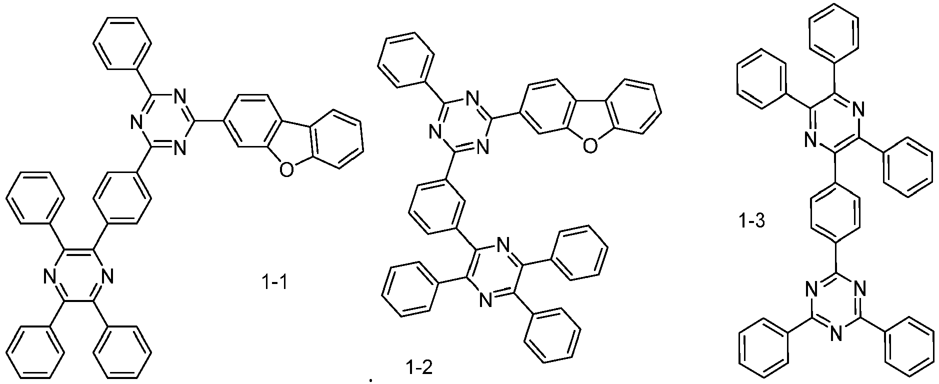

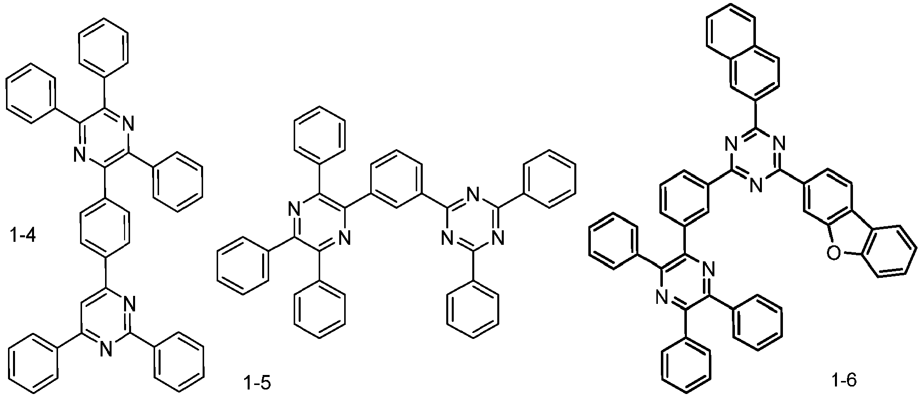

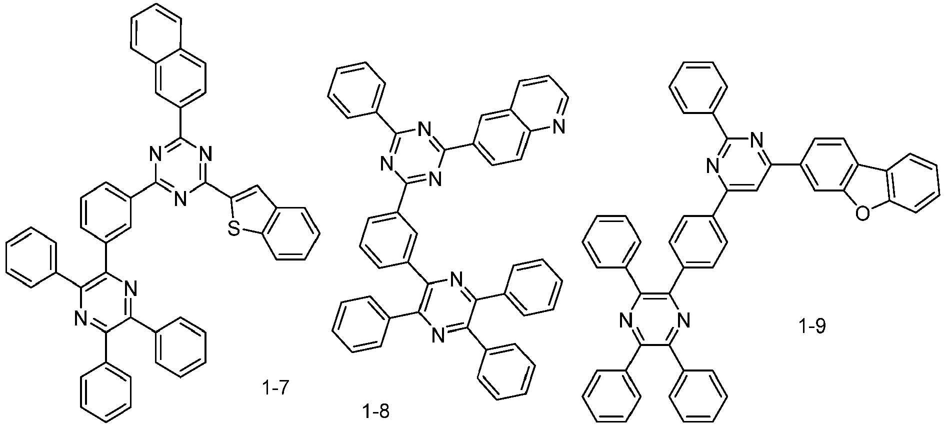

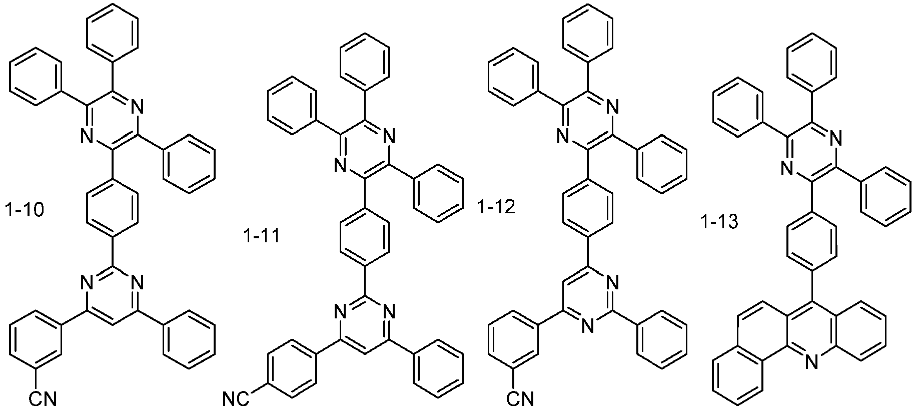

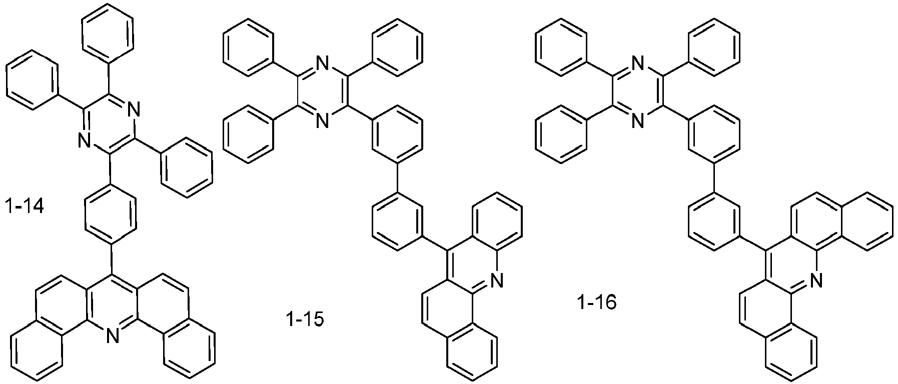

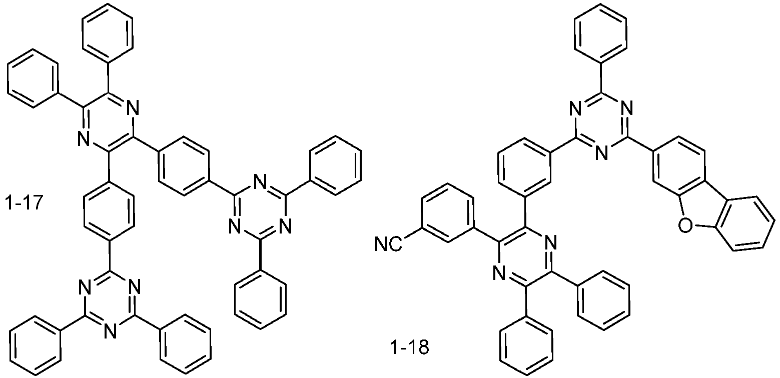

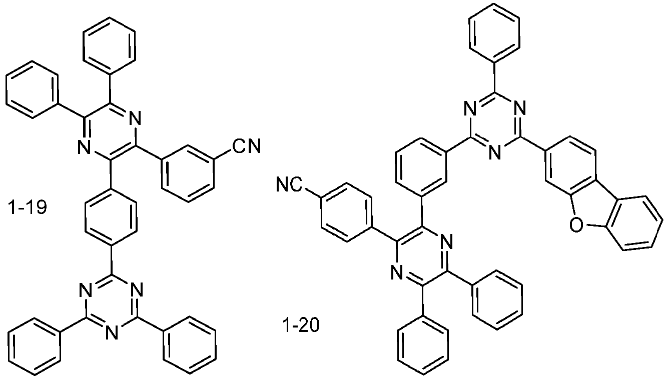

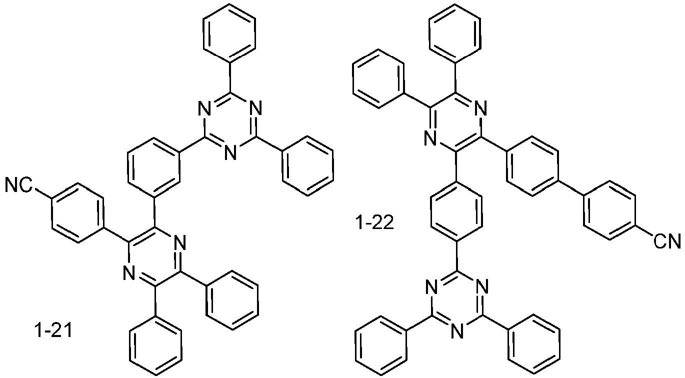

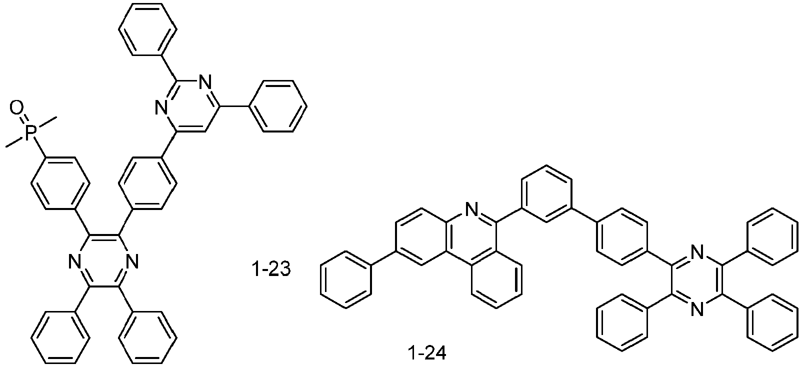

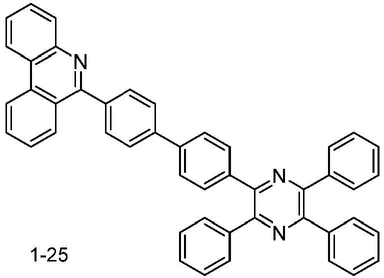

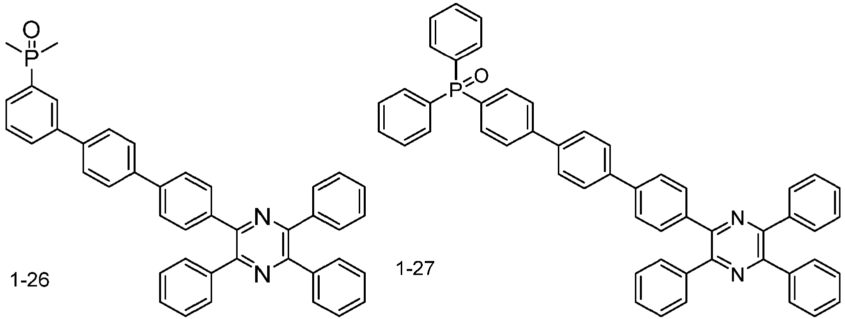

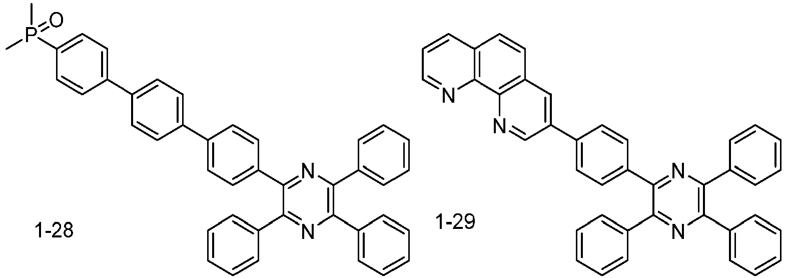

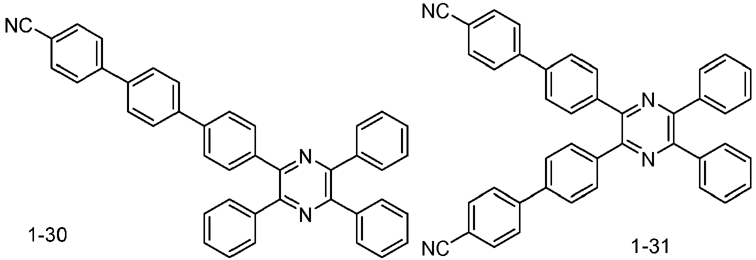

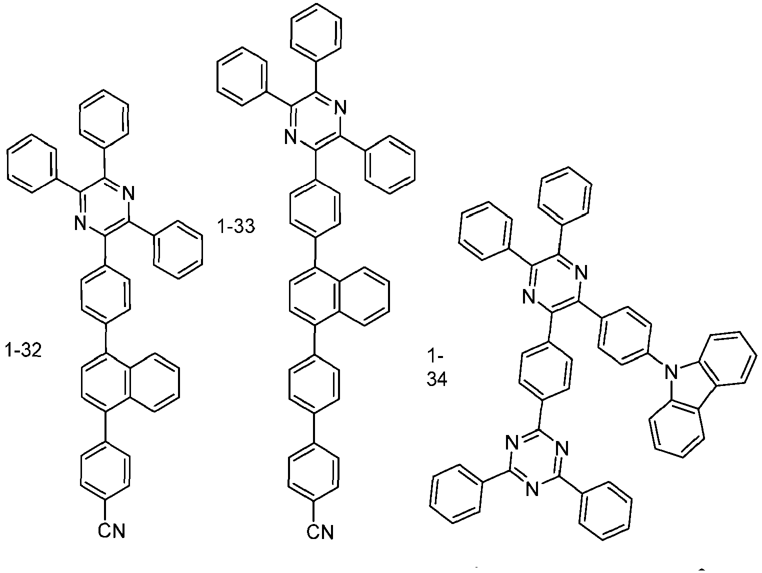

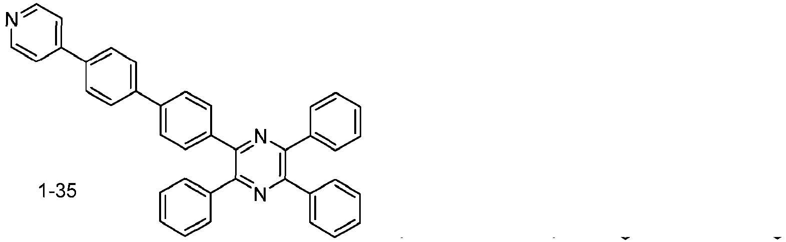

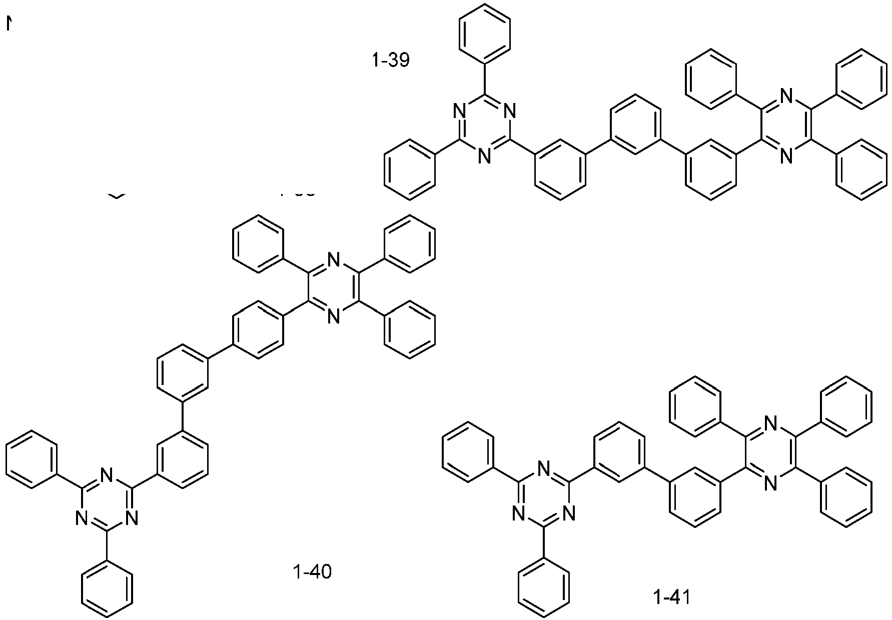

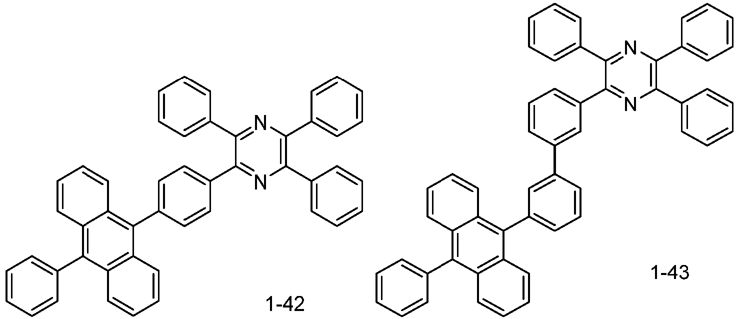

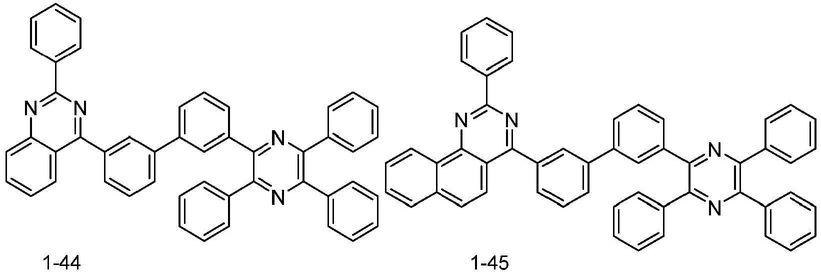

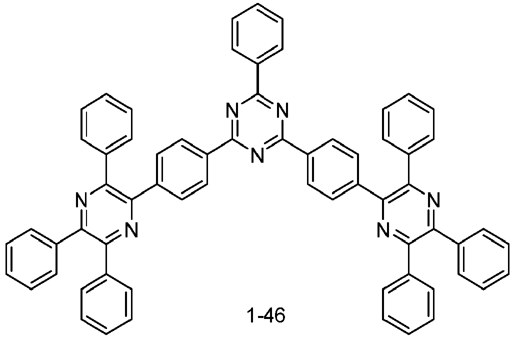

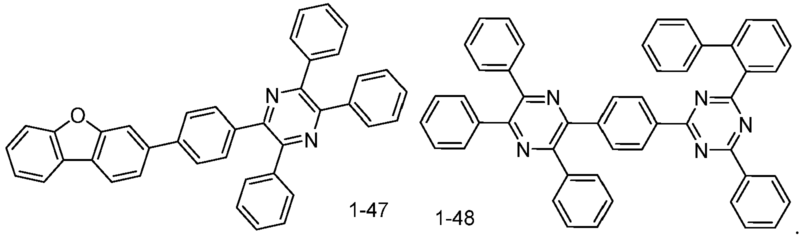

### Electrical n-dopant

Under electrical n-dopant, it is understood a compound which, if embedded into an electron transport matrix, improves, in comparison with the neat matrix under the same physical condictions, the electron properties of the formed organic material, particularly in terms of electron injection and/or electron conductivity.

In the context of the present invention "embedded into an electron transport matrix" means homogenously mixed with the electron transport matrix.

The electrical n-dopant may be selected from elemental metals, metal salts, metal complexes and organic radicals.

In one embodiment, the electrical n-dopant is selected from alkali metal salts and alkali metal complexes; preferably from lithium salts and lithium organic complexes; more preferably from lithium halides and lithium organic chelates; even more preferably from lithium fluoride, a lithium quinolinolate, lithium borate, lithium phenolate, lithium pyridinolate or from a lithium complex with a Schiff base ligand; most preferably,
- the lithium complex has the formula II, III or IV: 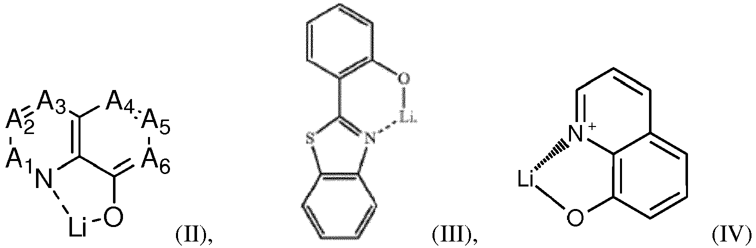 wherein
   A1 to A6 are same or independently selected from CH, CR, N, O;
   R is same or independently selected from hydrogen, halogen, alkyl or aryl or heteroaryl with 1 to 20 carbon atoms; and more preferred A1 to A6 are CH,
- the borate based organic ligand is a tetra(1H-pyrazol-1-yl)borate,
- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
- the pyridinolate is a 2-(diphenylphosphoryl)pyridin-3-olate,
- the lithium Schiff base has the structure 100, 101, 102 or 103:

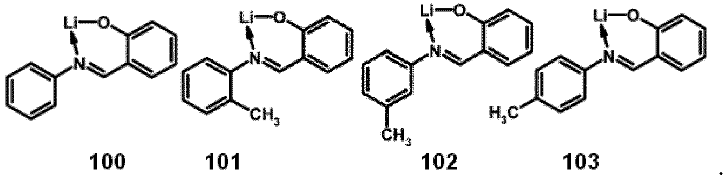

In another embodiment, the electrical n-dopant is a redox n-dopant.

### Redox n-dopant

Under redox n-dopant, it is understood a compound which, if embedded into an electron transport matrix, increases concentration of free electrons in comparison with the neat matrix under the same physical conditions.

The redox n-dopant may not emit light under the operation condition of an electroluminescent device, for example an OLED. In one embodiment, the redox n-dopant is selected from an elemental metal, an electrically neutral metal complex and/or an electrically neutral organic radical.

The most practical benchmark for the strength of an n-dopant is the value of its redox potential. There is no particular limitation in terms how negative the value of the redox potential can be.

As reduction potentials of usual electron transport matrices used in organic semiconductors are, if measured by cyclic voltammetry against ferrocene/ferrocenium reference redox couple, roughly in the range from about - 0.8 V to about - 3.1V; the practically applicable range of redox potentials for n-dopants which can effectively n-dope such matrices is in a slightly broader range, from about - 0.5 to about - 3.3 V.

The measurement of redox potentials is practically performed for a corresponding redox couple consisting of the reduced and of the oxidized form of the same compound. In case that the redox n-dopant is an electrically neutral metal complex and/or an electrically neutral organic radical, the measurement of its redox potential is actually performed for the redox couple formed by
(i) the electrically neutral metal complex and its cation radical formed by an abstraction of one electron from the electrically neutral metal complex, or
(ii) the electrically neutral organic radical and its cation formed by an abstraction of one electron from the electrically neutral organic radical.

Preferably, the redox potential of the electrically neutral metal complex and/or of the electrically neutral organic radical may have a value which is more negative than - 0.5 V, preferably more negative than - 1.2 V, more preferably more negative than - 1.7 V, even more preferably more negative than - 2.1 V, most preferably more negative than - 2.5 V, if measured by cyclic voltammetry against ferrocene/ferrocenium reference redox couple for a corresponding redox couple consisting of
(i) the electrically neutral metal complex and its cation radical formed by an abstraction of one electron from the electrically neutral metal complex, or
(ii) the electrically neutral organic radical and its cation formed by an abstraction of one electron from the electrically neutral organic radical.

In a preferred embodiment, the redox potential of the n-dopant is between the value which is about 0.5 V more positive and the value which is about 0.5 V more negative than the value of the reduction potential of the chosen electron transport matrix.
Electrically neutral metal complexes suitable as redox n-dopants may be e.g. strongly reductive complexes of some transition metals in low oxidation state. Particularly strong redox n-dopants may be selected for example from Cr(II), Mo(II) and/or W(II) guanidinate complexes such as W₂(hpp)₄, as described in more detail in WO2005/086251.

Electrically neutral organic radicals suitable as redox n-dopants may be e.g. organic radicals created by supply of additional energy from their stable dimers, oligomers or polymers, as described in more detail in EP 1 837 926 B1, WO2007/107306, or WO2007/107356. Under an elemental metal, it is understood a metal in a state of a neat metal, of a metal alloy, or in a state of free atoms or metal clusters. It is understood that metals deposited by vacuum thermal evaporation from a metallic phase, e.g. from a neat bulk metal, vaporize in their elemental form.

It is further understood that if the vaporized elemental metal is deposited together with a covalent matrix, the metal atoms and/or clusters are embedded in the covalent matrix. In other words, it is understood that any metal doped covalent material prepared by vacuum thermal evaporation contains the metal at least partially in its elemental form.

For the use in consumer electronics, only metals containing stable nuclides or nuclides having very long halftime of radioactive decay might be applicable. As an acceptable level of nuclear stability, the nuclear stability of natural potassium can be taken.

In one embodiment, the n-dopant may be selected from electropositive metals selected from alkali metals, alkaline earth metals, rare earth metals and metals of the first transition period Ti, V, Cr and Mn. Preferably, the n-dopant may be selected from_Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

The redox dopant may be essentially non-emissive.

According to another aspect of the invention, it is provided an electronic device comprising a first electrode, a second electrode, and arranged between the first and second electrode, a layer comprising the organic material according to invention. The layer of the organic material according to invention may serve as an electron transport layer and/or a hole blocking layer. In one embodiment, the electronic device is an electroluminescent device. Preferably, the electroluminescent device is an organic light emitting diode. In one embodiment, the layer comprising the compound according to invention further comprises an electrical dopant. In one embodiment, the electrical dopant comprised in the layer may be a n-dopant selected from elemental metals, metal complexes and metal salts. In one embodiment, the n-dopant may be selected from salts and/or complexes of alkali metals, alkaline earth metals, and transition metals, preferably from salts and/or complexes of Li, Na, K, Rb, Cs, Mg, Ca, Sr, Ba, Fe, Mn, Zn.

According to another aspect of the invention, it is provided an electronic device comprising at least one electroluminescent device according to any embodiment described throughout this application, preferably, the electronic device comprises the organic light emitting diode in one of embodiments described throughout this application. More preferably, the electronic device is a display device.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the subclaims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
FIG. 1 is a cross-sectional view showing an organic light emitting diode according to an embodiment of the invention.
FIGS. 2 and 3 are cross-sectional views specifically showing a part of an organic layer of an organic light emitting diode according to an embodiment of the invention.

Hereinafter, the figures are illustrated in more detail with reference to examples. However, the present disclosure is not limited to the following figures.

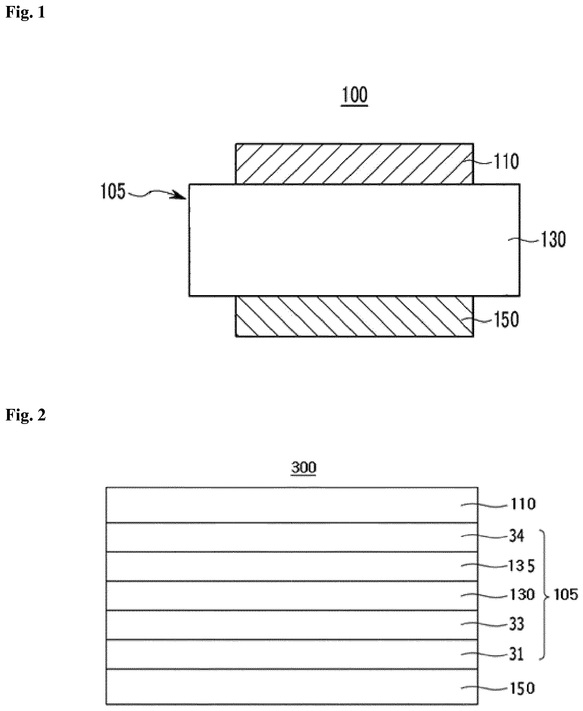

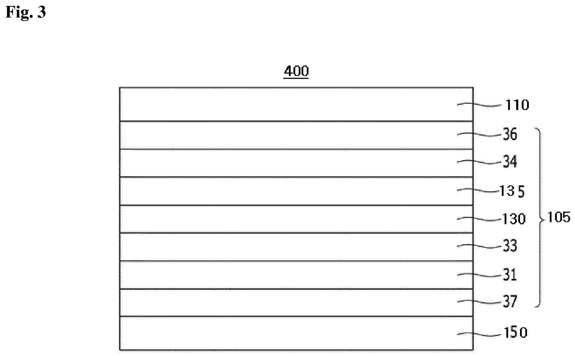

FIGS. 1 to 3 are schematic cross-sectional views of organic light emitting diodes 100, 300, and 400 according to an embodiment of the present invention. Hereinafter, referring to FIG 1, a structure of an organic light emitting diode according to an embodiment of the present invention and a method of manufacturing the same are as follows. The organic light emitting diode 100 has a structure where an anode 110, a stack of organic layers 105 including an optional hole transport region; an emission layer 130; and a cathode 150 that are sequentially stacked.

A substrate may be disposed on the anode 110 or under the cathode 150. The substrate may be selected from usual substrate used in a general organic light emitting diode and may be a glass substrate or a transparent plastic substrate.

The anode 110 may be formed by depositing or sputtering an anode material on a substrate. The anode material may be selected from materials having a high work function that makes hole injection easy. The anode 110 may be a reflective electrode, a transflective electrode, or a transmissive electrode. The anode material may use indium tin oxide (ITO), indium zinc oxide (IZO), tin oxide (SnO₂), zinc oxide (ZnO), and the like. Or, it may be a metal such as silver (Ag), or gold (Au), or an alloy thereof.

The anode 110 may have a monolayer or a multi-layer structure of two or more layers.

The organic light emitting diodes 100, 300, and 400 according to an embodiment of the present invention may include a hole transport region; an emission layer 130; and a first electron transport layer 31 comprising a compound according to invention.

Referring to FIG. 2, the hole transport region of the stack of organic layers 105 may include at least two layered hole transport layers, and in this case, the hole transport layer contacting the emission layer (130) is defined as a second hole transport layer 135 and the hole transport layer contacting the anode (110) is defined as a first hole transport layer 34. The stack of organic layers 105 further includes two further layers, namely a hole blocking layer 33 and an electron transport layer 31. The hole transport region of the stack 105 may further include at least one of a hole injection layer, a hole transport layer, an electron blocking layer, and a buffer layer.

The hole transport region of the stack 105 may include only hole injection layer or only hole transport layer. Or, the hole transport region may have a structure where a hole injection layer 36/hole transport layer 34 or hole injection layer 36/hole transport layer 34/electron blocking layer (135) is sequentially stacked from the anode 110.

For example, the hole injection layer 36 and the electron injection layer 37 can be additionally included, so that an OLED may comprise an anode 110/hole injection layer 36/first hole transport layer 34/electron blocking layer 135/emission layer 130/hole blocking layer 33/ electron transport layer 31/electron injection layer 37/cathode 150, which are sequentially stacked.

According to another aspect of the invention, the organic electroluminescent device (400) comprises an anode (110), a hole injection layer (36), a first hole transport layer (34), optional an electron blocking layer (135), an emission layer (130), hole blocking layer (33), electron transport layer (31), an optional electron injection layer (37), a cathode (150) wherein the layers are arranged in that order.

The hole injection layer 36 may improve interface properties between ITO as an anode and an organic material used for the hole transport layer 34, and is applied on a non-planarized ITO and thus planarizes the surface of the ITO. For example, the hole injection layer 36 may include a material having a median value of the energy level of its highest occupied molecular orbital (HOMO) between the work function of ITO and the energy level of the HOMO of the hole transport layer 34, in order to adjust a difference between the work function of ITO as an anode and the energy level of the HOMO of the first hole transport layer 34.

When the hole transport region includes a hole injection layer 36, the hole injection layer may be formed on the anode 110 by any of a variety of methods, for example, vacuum deposition, spin coating, casting, Langmuir-Blodgett (LB) method, or the like.

When hole injection layer is formed using vacuum deposition, vacuum deposition conditions may vary depending on the material that is used to form the hole injection layer, and the desired structure and thermal properties of the hole injection layer to be formed and for example, vacuum deposition may be performed at a temperature of about 100 °C to about 500 °C, a pressure of about 10⁻⁶ Pa to about 10⁻¹ Pa, and a deposition rate of about 0.1 to about 10 nm/sec, but the deposition conditions are not limited thereto.

When the hole injection layer is formed using spin coating, the coating conditions may vary depending on the material that is used to form the hole injection layer, and the desired structure and thermal properties of the hole injection layer to be formed. For example, the coating rate may be in the range of about 2000 rpm to about 5000 rpm, and a temperature at which heat treatment is performed to remove a solvent after coating may be in a range of about 80 °C to about 200 °C, but the coating conditions are not limited thereto.

Conditions for forming the hole transport layer and the electron blocking layer may be defined based on the above-described formation conditions for the hole injection layer.

A thickness of the hole transport part of the charge transport region may be from about 10 nm to about 1000 nm, for example, about 10 nm to about 100 nm. When the hole transport part of the charge transport region includes the hole injection layer and the hole transport layer, a thickness of the hole injection layer may be from about 10 nm to about 1000 nm, for example about 10 nm to about 100 nm and a thickness of the hole transport layer may be from about 5 nm to about 200 nm, for example about 10 nm to about 150 nm. When the thicknesses of the hole transport part of the charge transport region, the HIL, and the HTL are within these ranges, satisfactory hole transport characteristics may be obtained without a substantial increase in driving voltage.

Hole transport matrix materials used in the hole transport region are not particularly limited. Preferred are covalent compounds comprising a conjugated system of at least 6 delocalized electrons. The term "covalent compound" is in more detail explained below, in the paragraph regarding the second electron transport matrix. Typical examples of hole transport matrix materials which are widely used in hole transport layers are polycyclic aromatic hydrocarbons, triaryl amine compounds and heterocyclic aromatic compounds. Suitable ranges of frontier orbital energy levels of hole transport matrices useful in various layer of the hole transport region are well-known. In terms of the redox potential of the redox couple HTL matrix/ cation radical of the HTL matrix, the preferred values (if measured for example by cyclic voltammetry against ferrocene/ferrocenium redox couple as reference) may be in the range 0.0 - 1.0 V, more preferably in the range 0.2 - 0.7 V, even more preferably in the range 0.3 - 0.5 V.

The hole transport region of the stack of organic layers may further include a charge-generating material to improve conductivity, in addition to the materials as described above. The charge-generating material may be homogeneously or non-homogeneously dispersed in the hole transport region.

The charge-generating material may be, for example, a p-dopant. The p-dopant may be one of a quinone derivative, a metal oxide, and a cyano group-containing compound, but is not limited thereto. Non-limiting examples of the p-dopant are quinone derivatives such as tetracyanoquinonedimethane (TCNQ), 2,3,5,6-tetrafluoro-tetracyano-1,4-benzoquinonedimethane (F4-TCNQ), and the like; metal oxides such as tungsten oxide, molybdenum oxide, and the like; and cyano-containing compounds such as compound IIT-D1 below.

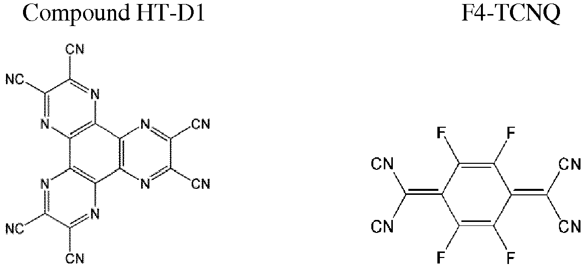

The hole transport part of the charge transport region may further include a buffer layer.

The buffer layer may compensate for an optical resonance distance of light according to a wavelength of the light emitted from the EML, and thus may increase efficiency.

The emission layer (EML) may be formed on the hole transport region by using vacuum deposition, spin coating, casting, LB method, or the like. When the emission layer is formed using vacuum deposition or spin coating, the conditions for deposition and coating may be similar to those for the formation of the hole injection layer, though the conditions for the deposition and coating may vary depending on the material that is used to form the emission layer. The emission layer may include an emitter host (EML host) and an emitter dopant (further only emitter).

The emitter may be a red, green, or blue emitter.

In one embodiment, the emitter host is an anthracene matrix compound represented by formula 400 below:

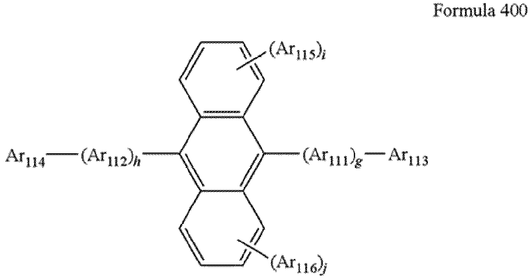

In formula 400, Arm and Ar₁₁₂ may be each independently a substituted or unsubstituted C₆-C₆₀ arylene group; Ar₁₁₃ to Ar₁₁₆ may be each independently a substituted or unsubstituted C₁-C₁₀ alkyl group or a substituted or unsubstituted C₆-C₆₀ aryl group; and g, h, i, and j may be each independently an integer from 0 to 4. In some embodiments, Ar₁₁₁ and Ar₁₁₂ in formula 400 may be each independently one of a phenylene group, a naphthylene group, a phenanthrenylene group, or a pyrenylene group; or a phenylene group, a naphthylene group, a phenanthrenylene group, a fluorenyl group, or a pyrenylene group, each substituted with at least one of a phenyl group, a naphthyl group, or an anthryl group.

In formula 400, g, h, i, and j may be each independently an integer of 0, 1, or 2. In formula 400, Ar₁₁₃ to Ar₁₁₆ may be each independently one of
- a C₁-C₁₀ alkyl group substituted with at least one of a phenyl group, a naphthyl group, or an anthryl group;
- a phenyl group, a naphthyl group, an anthryl group, a pyrenyl group, a phenanthrenyl group, or a fluorenyl group;
- a phenyl group, a naphthyl group, an anthryl group, a pyrenyl group, a phenanthrenyl group, or
- a fluorenyl group, each substituted with at least one of a deuterium atom, a halogen atom, a hydroxyl group, a cyano group, a nitro group, an amino group, an amidino group, a hydrazine group, a hydrazone group, a carboxyl group or a salt thereof,
- a sulfonic acid group or a salt thereof,
- a phosphoric acid group or a salt thereof,
- a C₁-C₆₀ alkyl group, a C₂-C₆₀ alkenyl group, a C₂-C₆₀ alkynyl group, a C₁-C₆₀ alkoxy group, a phenyl group, a naphthyl group, an anthryl group, a pyrenyl group, a phenanthrenyl group, or
- a fluorenyl group; or 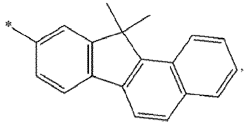 or formulas (Y2) or (Y3):

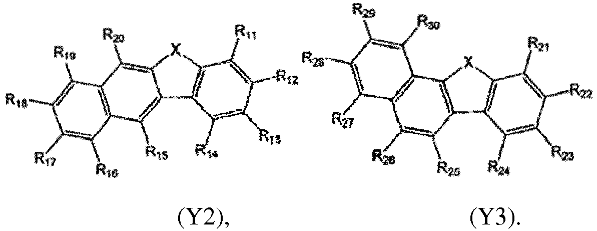

Wherein in the formulas (Y2) and (Y3), X is selected form an oxygen atom and a sulfur atom, but embodiments of the invention are not limited thereto.

In the formula (Y2), any one of R₁₁ to R₁₄ is used for bonding to Ar₁₁₁. R₁₁ to R₁₄ that are not used for bonding to Arm and R₁₅ to R₂₀ are the same as R₁ to R₈.

In the formula (Y3), any one of R₂₁ to R₂₄ is used for bonding to Ar₁₁₁. R₂₁ to R₂₄ that are not used for bonding to Arm and R₂₅ to R₃₀ are the same as R₁ to R₈.

Preferably, the EML host comprises between one and three heteroatoms selected from the group consisting of N, O or S. More preferred the EML host comprises one heteroatom selected from S or O.

According to a further aspect of the invention, the emitter host respectively has a reduction potential which, if measured under the same conditions by cyclic voltammetry against Fc/Fc⁺ in tetrahydrofuran, has a value more negative than the respective value obtained for 7-([1,1'-biphenyl]-4-yl)dibenzo[c,h]acridine, preferably more negative than the respective value for 9,9',10,10'-tetraphenyl-2,2'-bianthracene, more preferably more negative than the respective value for 2,9-di([1,1'-biphenyl]-4-yl)-4,7-diphenyl-1,10-phenanthroline, even more preferably more negative than the respective value for 2,4,7,9-tetraphenyl-1,10-phenanthroline, even more preferably more negative than the respective value for 9,10-di(naphthalen-2-yl)-2-phenylanthracene, even more preferably more negative than the respective value for 2,9-bis(2-methoxyphenyl)-4,7-diphenyl-1,10-phenanthroline, most preferably more negative than the respective value for 9,9'-spirobi[fluorene]-2,7-diylbis(diphenylphosphine oxide).

The emitter is mixed in a small amount to cause light emission, and may be generally a material such as a metal complex that emits light by multiple excitation into a triplet or more. The emitter may be, for example an inorganic, organic, or organometallic compound, and one or more kinds thereof may be used.

The emitter may be a fluorescent emitter, for example ter-fluorene, the structures are shown below. 4.4'-bis(4-diphenyl amiostyryl)biphenyl (DPAVBi), 2,5,8,11-tetra-tert-butyl perylene (TBPe), and Compound 4 below are examples of fluorescent blue emitters.

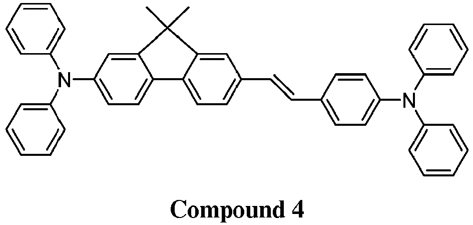

According to another aspect, the organic semiconductor layer comprising a compound according to invention is arranged between a fluorescent blue emission layer and the cathode electrode.

The emitter may be a phosphorescent emitter, and examples of the phosphorescent emitters may be organometallic compounds including Ir, Pt, Os, Ti, Zr, Hf, Eu, Tb, Tm, Fe, Co, Ni, Ru, Rh, Pd, or a combination thereof. The phosphorescent emitter may be, for example a compound represented by formula Z, but is not limited thereto:

L₂MX (Z).

In formula Z, M is a metal, and L and X are the same or different, and are a ligand to form a complex compound with M.

The M may be, for example Ir, Pt, Os, Ti, Zr, Hf, Eu, Tb, Tm, Fe, Co, Ni, Ru, Rh, Pd or, in a polynuclear complex,a combination thereof, and the L and X may be, for example, a bidendate ligand.

A thickness of the emission layer may be about 10 nm to about 100 nm, for example about 20 nm to about 60 nm. When the thickness of the emission layer is within these ranges, the emission layer may have improved emission characteristics without a substantial increase in a driving voltage.

Next, the electron transport region of the stack of organic layers 105 is disposed on the emission layer.

The electron transport region of the stack of organic layers includes at least an electron transport layer. The electron transport region of the stack of organic layers may further include an electron injection layer and/or a hole blocking layer. At least an electron transport layer comprises the n-doped organic material according to one of its various embodiments.

For example, the electron transport region of the stack of organic layers may have a structure of an electron transport layer/hole blocking layer/electron injection layer but is not limited thereto. For example, an organic light emitting diode according to an embodiment of the present invention includes at least two electron transport layers in the electron transport region of the stack of organic layers 105, and in this case, the layer contacting the emission layer is a hole blocking layer 33.

The electron transport layer may include two or more different electron transport matrix compounds.

### Second electron transport matrix compound

Various embodiments of the electron transport region in the device according to invention, e.g. devices comprising hole blocking layers, electron injecting layers, may comprise a second electron transport matrix compound.

Second electron transport matrix is not particularly limited. Similarly as other materials which are in the inventive device comprised outside the emitting layer, the second electron transport matrix may not emit light.

According to one embodiment, the second electron transport matrix can be an organic compound, an organometallic compound, or a metal complex.

According to one embodiment, the second electron transport matrix may be a covalent compound comprising a conjugated system of at least 6 delocalized electrons. Under a covalent material in a broadest possible sense, it might be understood a material, wherein at least 50 % of all chemical bonds are covalent bonds, wherein coordination bonds are also considered as covalent bonds. In the present application, the term encompasses in the broadest sense all usual electron transport matrices which are predominantly selected from organic compounds but also e.g. from compounds comprising structural moieties which do not comprise carbon, for example substituted 2,4,6-tribora-1,3,5 triazines, or from metal complexes, for example aluminium tris(8-hydroxyquinolinolate).

The molecular covalent materials can comprise low molecular weight compounds which may be, preferably, stable enough to be processable by vacuum thermal evaporation (VTE). Alternatively, covalent materials can comprise polymeric covalent compounds, preferably, compounds soluble in a solvent and thus processable in form of a solution. It is to be understood that a polymeric substantially covalent material may be crosslinked to form an infinite irregular network, however, it is supposed that such crosslinked polymeric substantially covalent matrix compound still comprises both skeletal as well as peripheral atoms. Skeletal atoms of the covalent compound are covalently bound to at least two neighbour atoms. Other atoms of the covalent compound are peripheral atoms which are covalently bound with a single neighbour atom. Inorganic infinite crystals or fully crosslinked networks having partly covalent bonding but substantially lacking peripheral atoms, like silicon, germanium, gallium arsenide, indium phosphide, zinc sulfide, silicate glass etc. are not considered as covalent matrices in the sense of present application, because such fully crosslinked covalent materials comprise peripheral atoms only on the surface of the phase formed by such material. A compound comprising cations and anions is still considered as covalent, if at least the cation or at least the anion comprises at least ten covalently bound atoms.

Preferred examples of covalent second electron transport matrix compounds are organic compounds, consisting predominantly from covalently bound C, H, O, N, S, which may optionally comprise also covalently bound B, P, As, Se. In one embodiment, the second electron transport matrix compound lacks metal atoms and majority of its skeletal atoms is selected from C, O, S, N.

In another embodiment, the second electron transport matrix compound comprises a conjugated system of at least six, more preferably at least ten, even more preferably at least fourteen delocalized electrons.

Examples of conjugated systems of delocalized electrons are systems of alternating pi- and sigma bonds. Optionally, one or more two-atom structural units having the pi-bond between its atoms can be replaced by an atom bearing at least one lone electron pair, typically by a divalent atom selected from O, S, Se, Te or by a trivalent atom selected from N, P, As, Sb, Bi. Preferably, the conjugated system of delocalized electrons comprises at least one aromatic or heteroaromatic ring adhering to the Hückel rule. Also preferably, the second electron transport matrix compound may comprise at least two aromatic or heteroaromatic rings which are either linked by a covalent bond or condensed.

In one of specific embodiments, the second electron transport matrix compound comprises a ring consisting of covalently bound atoms and at least one atom in the ring is phosphorus.

In a more preferred embodiment, the phosphorus-containing ring consisting of covalently bound atoms is a phosphepine ring.

In another preferred embodiment, the covalent matrix compound comprises a phosphine oxide group. Also preferably, the substantially covalent matrix compound comprises a heterocyclic ring comprising at least one nitrogen atom. Examples of nitrogen containing heterocyclic compounds which are particularly advantageous as second electron transport matrix compound for the inventive device are matrices comprising, alone or in combination, pyridine structural moieties, diazine structural moieties, triazine structural moieties, quinoline structural moieties, benzoquinoline structural moieties, quinazoline structural moieties, acridine structural moieties, benzacridine structural moieties, dibenzacridine structural moieties, diazole structural moieties and benzodiazole structural moieties.

The second matrix compound may have a molecular weight (Mw) of ≥ 400 to ≤ 850 g / mol, preferably ≥ 450 to ≤ 830 g / mol. If the molecular weight is selected in this range, particularly reproducible evaporation and deposition can be achieved in vacuum at temperatures where good long-term stability is observed.

Preferably, the second matrix compound may be essentially non-emissive.

According to another aspect, the reduction potential of the second electron transport compound may be selected more negative than -2.2 V and less negative than -2.35 V against Fc/Fc⁺ in tetrahydrofuran, preferably more negative than -2.25 V and less negative than -2.3 V.

According to one embodiment, the first and the second matrix compound may be selected different, and
- the second electron transport layer consist of a second matrix compound; and
- the first electron transport layer consist of the organic material of according to invention, and an electrical n-dopant, preferably an alkali metal salt or an alkali metal organic complex.

Preferably, the first and second electron transport layer may be essentially non-emissive.

According to one embodiment the hole blocking layer may comprise the organic material.

According to another embodiment, the second electron transport layer can be in direct contact with the emission layer.

According to another embodiment, the electron transport layer can be in direct contact with a hole blocking layer.

According to another embodiment, the second electron transport layer can be contacting sandwiched between the emission layer and the first electron transport layer.

According to another embodiment, the first electron transport layer can be in direct contact with the electron injection layer.

According to another embodiment, the first electron transport layer can be contacting sandwiched between the second electron transport layer and the electron injection layer.

According to another embodiment, the first electron transport layer can be in direct contact with the cathode electrode.

According to another embodiment, the first electron transport layer can be contacting sandwiched between the second electron transport layer and the cathode layer.

According to another embodiment, the second electron transport layer can be contacting sandwiched between the emission layer and the first electron transport layer, and the first electron transport layer can be contacting sandwiched between the second electron transport layer and the electron injection layer or sandwiched between the second electron transport layer and the hole blocking layer.

According to another embodiment, the second electron transport layer may be formed of the compound according to invention.

The formation conditions of the first electron transport layer 31, hole blocking layer 33, and electron injection layer 37 of the electron transport region of the stack of organic layers refer to the formation conditions of the hole injection layer.

The thickness of the first electron transport layer may be from about 2 nm to about 100 nm, for example about 3 nm to about 30 nm. When the thickness of the first electron transport layer is within these ranges, the first electron transport layer may have improved electron transport auxiliary ability without a substantial increase in driving voltage.

A thickness of the second electron transport layer may be about 10 nm to about 100 nm, for example about 15 nm to about 50 nm. When the thickness of the electron transport layer is within these ranges, the electron transport layer may have satisfactory electron transporting ability without a substantial increase in driving voltage.

According to another aspect of the invention, the organic electroluminescent device further comprises an electron injection layer between the second electron transport layer and the cathode.

The electron injection layer (EIL) 37 may facilitate injection of electrons from the cathode 150.

According to another aspect of the invention, the electron injection layer 37 comprises:
(i) an electropositive metal selected from alkali metals, alkaline earth metals and rare earth metals in substantially elemental form, preferably selected from Li, Na, K, Rb, Cs, Mg, Ca, Sr, Ba, Eu and Yb, more preferably from Li, Na, Mg, Ca, Sr and Yb, even more preferably from Li and Yb, most preferably Yb; and/or
(ii) an alkali metal complex and/or alkali metal salt, preferably the Li complex and/or salt,
more preferably a Li quinolinolate, even more preferably a lithium 8-hydroxyquinolinolate, most preferably the alkali metal salt and/or complex of the second electron transport layer is idencial with the alkali metal salt and/or complex of the injection layer.

The electron injection layer may include at least one selected from LiF, NaCl, CsF, Li₂O, and BaO.

A thickness of the EIL may be from about 0.1 nm to about 10 nm, or about 0.3 nm to about 9 nm. When the thickness of the electron injection layer is within these ranges, the electron injection layer may have satisfactory electron injection ability without a substantial increase in driving voltage.

A material for the cathode 150 may be a metal, an alloy, or an electrically conductive compound that has a low work function, or a combination thereof. Specific examples of the material for the cathode 150 may be lithium (Li), magnesium (Mg), aluminum (Al), aluminum-lithium (Al-Li), calcium (Ca), magnesium-indium (Mg-In), magnesium-silver (Mg-Ag), etc. In order to manufacture a top-emission light-emitting device having a reflective anode 110 deposited on a substrate, the cathode 150 may be formed as a transparent or translucent electrode from, for example, indium tin oxide (ITO) or indium zinc oxide (IZO).

In one embodiment, the organic electronic device according to the invention comprising an organic semiconducting layer comprising a compound according to invention can further comprise a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection layer, hole transporting and/or a hole generation layer the later one having the function of generating holes in a charge-generation layer or a p-n-junction.

In one embodiment, the radialene compound may have formula (XX) and/or the quinodimethane compound may have formula (XXIa) or (XXIb): 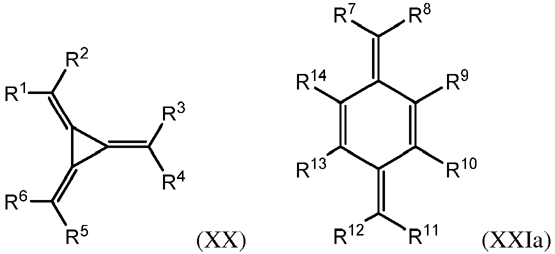 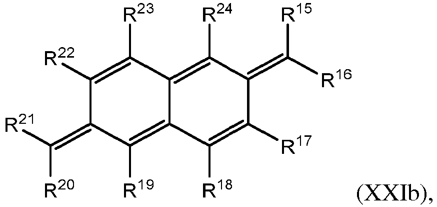 wherein R¹, R², R³, R⁴, R⁵, R⁶, R⁷, R⁸, R¹¹, R¹², R¹⁵, R¹⁶, R²⁰, R²¹ are independently selected from above mentioned electron withdrawing groups and R⁹, R¹⁰, R¹³, R¹⁴, R¹⁷, R¹⁸, R¹⁹, R²², R²³ and R²⁴ are independently selected from H, halogen and above mentioned electron withdrawing groups.

Hereinafter, the embodiments are illustrated in more detail with reference to examples. However, the present disclosure is not limited to the following examples.

### Detailed description

The invention is furthermore illustrated by the following examples which are illustrative only and non-binding.

### Building block preparation

### A. 2,3,5-triphenyl-6-(4-(4,4,5,5-tetramethyl-1,3,2-dioxaborolan-2-yl)phenyl)pyrazine

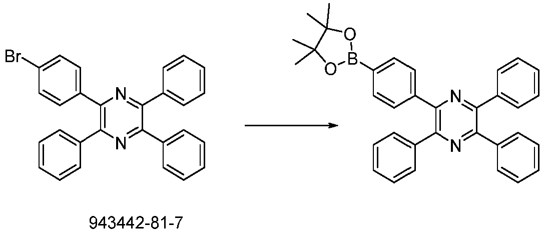

A flask was flushed with nitrogen and charged with 2-(4-bromophenyl)-3,5,6-triphenylpyrazine (15.0 g, 32.4 mmol), bis(pinacolato)diboron (9.04 g, 35.6 mmol), Pd(dppf)Cl₂ (0.71 g, 0.97 mmol), and potassium acetate (9.5 g, 97.2 mmol). Dry and deaerated DMF (90 mL) was added and the reaction mixture was heated to 100 °C under a nitrogen atmosphere overnight. Subsequently, all volatiles were removed in vacuo, water (400 mL) and dichloromethane (1 L) were added and the organic phase was washed with water (3 x 400 mL). After drying over MgSO₄, the organic phase was filtered through a pad of silica gel. After rinsing with additional dichloromethane (1 L), the filtrate was concentrated under reduced pressure to a minimal volume. Methanol (350 mL) was added and the suspension was left stirring at room temperature overnight. The solid was collected by suction filtration to yield 15.9 g (96%) of a white solid after drying.

### B. 2,3,5-triphenyl-6-(3-(4,4,5,5-tetramethyl-1,3,2-dioxaborolan-2-yl)phenyl)pyrazine

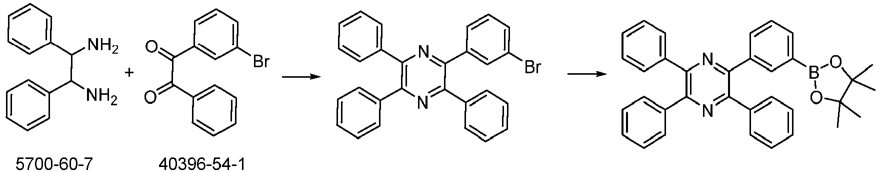

### Sa) 2-(3-bromophenyl)-3,5,6-triphenylpyrazine

A flask was charged with 1-(3-bromophenyl)-2-phenylethane-1,2-dione (36.6 g, 126.5 mmol), 1,2-diphenylethane-1,2-diamine (38.7 g, 182.1 mmol), and acetic acid (320) mL. The mixture was heated to 75 °C for 24 h. Subsequently, the reaction mixture was concentrated under reduced pressure to approx. 100 mL and then carefully poured into sat. aq. K₂CO₃ (700 mL). After extraction with dichloromethane three times, the combined organic phases were washed with brine, dried over MgSO₄, and concentrated under reduced pressure. The crude product was purified by column chromatography (silica, n-hexane/dichloromethane 8:2) and trituration with n-hexane to afford 38.8 g (66%) of a yellow solid after drying.

### Bb) 2,3,5-triphenyl-6-(3-(4,4,5,5-tetramethyl-1,3,2-dioxaborolan-2-yl)phenyl)pyrazine

Following the procedure described above for 2,3,5-triphenyl-6-(4-(4,4,5,5-tetramethyl-1,3,2-dioxaborolan-2-yl)phenyl)pyrazine using 2-(3-bromophenyl)-3,5,6-triphenylpyrazine (25 g, 54 mmol) afforded 22.5 g (82%) of a white solid.

### Preparation of compounds according to invention

### 2-(dibenzo[b,d]furan-3-yl)-4-phenyl-6-(4-(3,5,6-triphenylpyrazin-2-yl)phenyl)-1,3,5-triazine (1-1)

A flask was flushed with nitrogen and charged with (4-(3,5,6-triphenylpyrazin-2-yl)phenyl)boronic acid (13.9 g, 26.8 mmol), 2-chloro-4-(dibenzo[b,d]furan-3-yl)-6-phenyl-1,3,5-triazine (9.6 g, 26.8 mmol), Pd(PPh₃)₄ (1.5 g, 1.3 mmol), and K₂CO₃ (9.2 g, 66.9 mmol). A mixture of deaerated 1,4-Dioxane/water (5.7:1, 235 mL) was added and the reaction mixture was heated to 85 °C under a nitrogen atmosphere for two hours. Mixture was extracted in DCM/water and the combined organic phases were washed with brine and dried over MgSO₄. Solvent was removed partially, acetone (300 mL) was added. The resulting suspension was stirred at room temperature overnight and the precipitate was collected by suction filtration. The crude product was further purified by additional recrystallization from dichloromethane to yield 14.9 g (79%) of white solid after drying. Final purification was achieved by sublimation. HPLC: 99.8%, HPLC/ESI-MS m/z = 706 ([M+H]⁺)

### 2-(dibenzo[b,d]furan-3-yl)-4-phenyl-6-(3-(3,5,6-triphenylpyrazin-2-yl)phenyl)-1,3,5-triazine (1-2)

A flask was flushed with nitrogen and charged with 2,3,5-triphenyl-6-(3-(4,4,5,5-tetramethyl-1,3,2-dioxaborolan-2-yl)phenyl)pyrazine (5.0 g, 9.8 mmol), 2-chloro-4-(dibenzo[b,d]furan-3-yl)-6-phenyl-1,3,5-triazine (3.19 g, 8.91 mmol), Pd(PPh₃)₄ (0.2 g, 0.18 mmol), and K₂CO₃ (2.46 g, 17.8 mmol). A mixture of deaerated THF/water (4:1, 100 mL) was added and the reaction mixture was heated to 75 °C under a nitrogen atmosphere overnight. After cooling down to ambient temperature, the resulting precipitate was isolated by suction filtration and washed with THF. The crude product was then dissolved in hot chloroform and filtered through a pad of silica gel. After rinsing with additional hot chloroform, the filtrate was concentrated under reduced pressure to a minimal volume and n-hexane was added. The resulting precipitate was collected by suction filtration and washed with n-hexane. The crude product was further purified by recrystallization from toluene to yield 5.3 g (84%) of white solid after drying. Final purification was achieved by sublimation. HPLC/ESI-MS: 100%, m/z = 706 ([M+H]⁺)

### 2,4-diphenyl-6-(4-(3,5,6-triphenylpyrazin-2-yl)phenyl)-1,3,5-triazine (1-3)

A flask was flushed with nitrogen and charged with 2,3,5-triphenyl-6-(4-(4,4,5,5-tetramethyl-1,3,2-dioxaborolan-2-yl)phenyl)pyrazine (4.0 g, 7.8 mmol), 2-chloro-4,6-diphenyl-1,3,5-triazine (1.9 g, 7.1 mmol), Pd(PPh₃)₄ (0.16 g, 0.14 mmol), and K₂CO₃ (2.0 g, 14.0 mmol). A mixture of deaerated THF/water (4:1, 30 mL) was added and the reaction mixture was heated to 75 °C under a nitrogen atmosphere overnight. After cooling down to 5 °C, the resulting precipitate was isolated by suction filtration and washed with THF (1 × 5 mL) and n-hexane (3 x 5 mL). The crude product was then dissolved in dichloromethane (1 L) and the organic phase was washed with water (3 x 400 mL). After drying over MgSO₄, the organic phase was filtered through a pad of silica gel. After rinsing with additional dichloromethane (600 mL), the filtrate was concentrated under reduced pressure to a minimal volume and n-hexane (200 mL) was added. The resulting suspension was stirred at room temperature for 45 min and the precipitate was collected by suction filtration. The crude product was further purified by column chromatography (silica, n-hexane / dichloromethane 2:1 to n-hexane/ methanol 99:1) and recrystallization from chlorobenzene to yield 3.4 g (78%) of white solid after drying. Final purification was achieved by sublimation. HPLC/ESI-MS: 100%, m/z = 616 ([M+H]⁺)

### 2,4-diphenyl-6-(4-(3,5,6-triphenylpyrazin-2-yl)phenyl)pyrimidine (1-4)

A flask was flushed with nitrogen and charged with 2,3,5-triphenyl-6-(4-(4,4,5,5-tetramethyl-1,3,2-dioxaborolan-2-yl)phenyl)pyrazine (11.5 g, 22.5 mmol), 4-chloro-2,6-diphenylpyrimidine (6.0 g, 22.5 mmol), Pd(PPh₃)₄ (0.5 g, 0.5 mmol), and K₂CO₃ (6.2 g, 45.0 mmol). A mixture of deaerated THF/water (4:1, 100 mL) was added and the reaction mixture was heated to 75 °C under a nitrogen atmosphere overnight. After cooling down to room temperature, the resulting precipitate was isolated by suction filtration and washed with THF (2 x 10 mL) and n-hexane (3 x 15 mL). The crude product was then dissolved in dichloromethane (500 mL) and the organic phase was washed with water (3 x 300 mL). After drying over MgSO₄, the organic phase was filtered through a florisil pad. After rinsing with additional dichloromethane (500 mL), the filtrate was concentrated under reduced pressure to a minimal volume and n-hexane (500 mL) was added. The resulting suspension was stirred at room temperature for 30 min. The precipitate was collected by suction filtration and further purified by recrystallization from toluene to yield 9.2 g (67%) of a white solid after drying. Final purification was achieved by sublimation. HPLC/ESI-MS: 99.7%, m/z = 615 ([M+H]⁺)

### dimethyl(4"-(3,5,6-triphenylpyrazin-2-yl)-[1,1':4',1"-terphenyl]-4-yl)phosphine oxide (1-28)

### (4'-bromo-[1,1'-biphenyl]-4-yl)dimethylphosphine oxide

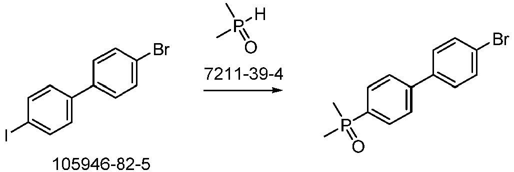

A flask was flushed with nitrogen and charged with 4-bromo-4'-iodo-1,1'-biphenyl (15 g, 41.8 mmol), dimethylphosphine oxide (5.22 g, 66.8 mmol), Pd₂(dba)₃ (0.38 g, 0.42 mmol), and Xantphos (0.48 g, 0.84 mmol). Dry and deaerated dioxane (220 mL) and triethylamine (7 mL) were added and the reaction mixture was stirred at ambient temperature for 48 h. Subsequently, the formed precipitate was isolated by suction filtration and washed with dioxane. After recrystallization from acetonitrile and drying, 2.15 g (17%) of an off-white solid were obtained.

### dimethyl(4"-(3,5,6-triphenylpyrazin-2-yl)-[1,1':4',1"-terphenyl]-4-yl)phosphine oxide (1-28)

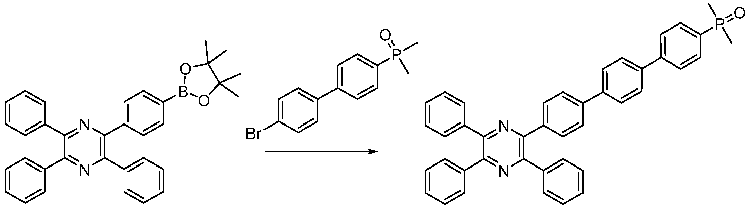

A flask was flushed with nitrogen and charged with 2,3,5-triphenyl-6-(4-(4,4,5,5-tetramethyl-1,3,2-dioxaborolan-2-yl)phenyl)pyrazine (3.2 g, 6.2 mmol), (4'-bromo-[1,1'-biphenyl]-4-yl)dimethylphosphine oxide (1.9 g, 6.2 mmol), Pd(PPh₃)₄ (0.14 g, 0.12 mmol), and K₂CO₃ (1.7 g, 12.4 mmol). A mixture of deaerated THF/water (4:1, 30 mL) was added and the reaction mixture was heated to 75 °C under a nitrogen atmosphere overnight. After cooling down to 5 °C, the resulting precipitate was isolated by suction filtration and washed with THF (2 x 5 mL) and n-hexane (3 x 5 mL). The crude product was then dissolved in dichloromethane (500 mL) and an aq. sodium diethylcarbamodithioate trihydrate solution (3%, 250 mL) was added. The mixture was stirred vigorously for 30 min and, subsequently, the organic phase was removed and further washed with water. After drying over MgSO₄, the organic phase was concentrated under reduced pressure to a minimal volume and n-hexane (200 mL) was added. The resulting suspension was stirred at room temperature for 1 h. The precipitate was collected by suction filtration and further purified by column chromatography (silica, dichloromethane/methanol 99:1 to dichloromethane/methanol 97:3). The pure fractions were combined and the solvents were removed under reduced pressure to afford 2.5 g (66%) of a white solid after drying. Final purification was achieved by sublimation. HPLC/ESI-MS: 100%, m/z = 613 ([M+H]⁺)

### 4"-(3,5,6-triphenylpyrazin-2-yl)-[1,1':4',1"-terphenyl]-4-carbonitrile (1-30)

A flask was flushed with nitrogen and charged with 2-(4-bromophenyl)-3,5,6-triphenylpyrazine (5.8 g, 12.5 mmol), 4'-(4,4,5,5-tetramethyl-1,3,2-dioxaborolan-2-yl)-[1,1'-biphenyl]-4-carbonitrile (5.0 g, 12.5 mmol), Pd(PPh₃)₄ (0.3 g, 0.25 mmol), and K₂CO₃ (3.4 g, 24.9 mmol). A mixture of deaerated 1,4-THF/water (4:1, 137.5 mL) was added and the reaction mixture was heated to 75 °C under a nitrogen atmosphere overnight. (4-(3,5,6-triphenylpyrazin-2-yl)phenyl)boronic acid (1.2 g, 2.3 mmol) was added and the reaction mixture was was heated to 75 °C under a nitrogen atmosphere overnight. Mixture was extracted in DCM/water and the combined organic phases were washed with brine. After drying over MgSO₄, the organic phase was filtered through a pad of silica gel. After rinsing with additional dichloromethane (500 mL), the filtrate was concentrated (80 mL) and hexane (1000 mL) was added. The resulting suspension was stirred at room temperature during an hour and the precipitate was collected by suction filtration. The crude product was further purified by additional recrystallization from dimethyformamide (3 x 25 mL) to yield 5.1 g (73%) of white solid after drying. Final purificatio n was achieved by sublimation. HPLC: 100%, HPLC/ESI-MS: m/z = 562 ([M+H]⁺)

### 2,4-diphenyl-6-(3"-(3,5,6-triphenylpyrazin-2-yl)-[1,1':3',1"-terphenyl]-3-yl)-1,3,5-triazine (1-39)

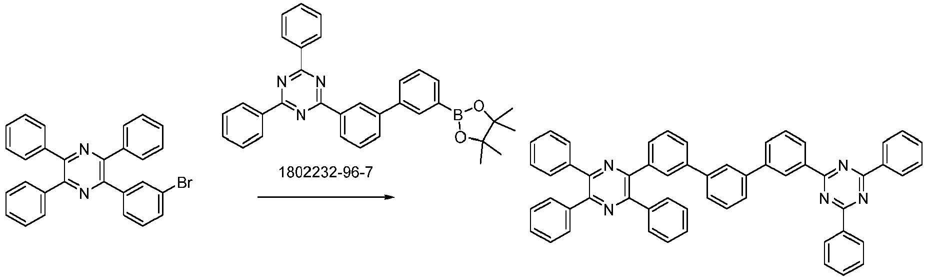

A flask was flushed with nitrogen and charged with 2-(3-bromophenyl)-3,5,6-triphenylpyrazine (4.76 g, 10.26 mmol), 2,4-diphenyl-6-(3'-(4,4,5,5-tetramethyl-1,3,2-dioxaborolan-2-yl)-[1,1'-biphenyl]-3-yl)-1,3,5-triazine (5.0 g, 9.78 mmol), Pd(dppf)Cl2 (0.14 g, 0.2 mmol), and K₂CO₃ (2.7 g, 19.6 mmol). A mixture of deaerated THF/water (5: 1, 60 mL) was added and the reaction mixture was heated to 75 °C under a nitrogen atmosphere overnight. After cooling down to ambient temperature, the resulting precipitate was isolated by suction filtration and washed with n-hexane. The crude product was then dissolved in chloroform and the organic phase was washed with water three times. The organic phase was concentrated under reduced pressure to a minimal volume and n-hexane was added. The resulting precipitate was collected by suction filtration and washed with n-hexane. The crude product was further purified by trituration with chloroform/n-hexane 1:5, column chromatography (silica, dichloromethane/n-hexane 1:1), and trituration with ethanol to yield 4.17 g (56%) of a white solid after drying. Final purification was achieved by sublimation. HPLC/ESI-MS: 100%, m/z = 768 ([M+H]⁺)

### 2-(4-(dibenzo[b,d]furan-3-yl)phenyl)-3,5,6-triphenylpyrazine (1-47)

A flask was flushed with nitrogen and charged with 2-(4-bromophenyl)-3,5,6-triphenylpyrazine (4.0 g, 8.6 mmol), dibenzo[b,d]furan-3-ylboronic acid (2.0 g, 9.5 mmol), Pd(PPh₃)₄ (0.2 g, 0.17 mmol), and K₂CO₃ (2.4 g, 17.3 mmol). A mixture of deaerated THF/water (4:1, 30 mL) was added and the reaction mixture was heated to 75 °C under a nitrogen atmosphere overnight. After cooling down to 5 °C, the resulting precipitate was isolated by suction filtration and washed with THF (2 x 4 mL) and n-hexane (3 x 5 mL). The crude product was then dissolved in dichloromethane (1 L) and the organic phase was washed with water (3 x 400 mL). After drying over MgSO₄, the organic phase was filtered through a pad of silica gel. After rinsing with additional dichloromethane (400 mL), the filtrate was concentrated under reduced pressure to a minimal volume and n-hexane (25 mL) was added. The resulting suspension was stirred at room temperature for 10 min and the precipitate was collected by suction filtration. The crude product was recrystallized from toluene to yield 3.6 g (76%) of a white solid after drying. Final purification was achieved by sublimation. HPLC/ESI-MS: 100%, m/z = 551 ([M+H]⁺)

### 2-([1,1'-biphenyl]-2-yl)-4-phenyl-6-(4-(3,5,6-triphenylpyrazin-2-yl)phenyl)-1,3,5-triazine (1-48)

A flask was flushed with nitrogen and charged with 2,3,5-triphenyl-6-(4-(4,4,5,5-tetramethyl-1,3,2-dioxaborolan-2-yl)phenyl)pyrazine (5.0 g, 9.8 mmol), 2-([1,1'-biphenyl]-2-yl)-4-chloro-6-phenyl-1,3,5-triazine (3.4 g, 9.8 mmol), Pd(PPh₃)₄ (0.23 g, 0.2 mmol), and K₂CO₃ (2.7 g, 19.6 mmol). A mixture of deaerated 1,4-THF/water (4:1, 110 mL) was added and the reaction mixture was heated to 75 °C under a nitrogen atmosphere overnight. Mixture was extracted in DCM/water and the combined organic phases were washed with brine. After drying over MgSO₄, the organic phase was filtered through a pad of silica gel. After rinsing with additional dichloromethane, solvents were removed under reduced pressure and the resulting solid was recrystallized in toluene (2 x 500 mL). The crude product was further purified by additional recrystallization from cholobenzene (125 mL) to yield 5.5 g (81 %) of white solid after drying. Final purification was achieved by sublimation. HPLC: 99.9%, HPLC/ESI-MS m/z = 692 ([M+H]⁺)

### Device example 1 (top emission blue OLED)

A glass substrate was cut to a size of 50 mm x 50 mm x 0.7 mm, ultrasonically cleaned with isopropyl alcohol for 5 minutes and then with pure water for 5 minutes, and cleaned again with UV ozone for 30 minutes, to prepare a first electrode. 100 nm Ag were deposited as anode at a pressure of 10⁻⁵ to 10⁻⁷ mbar to form the anode.

Then, 92 vol.-% auxiliary compound F1 (biphenyl-4-yl(9,9-diphenyl-9H-fluoren-2-yl)-[4-(9-phenyl-9H-carbazol-3-yl)phenyl]-amine, CAS 1242056-42-3) with 8 vol.-% auxiliary compound PD1 (2,2',2"-(cyclopropane-1,2,3-triylidene)tris(2-(p-cyanotetrafluorophenyl)acetonitrile) was vacuum deposited on the ITO electrode, to form a HIL having a thickness of 10 nm. Then, neat F1 was vacuum deposited on the HIL, to form a HTL having a thickness of 118 nm.

Then, F2 (N,N-bis(4-(dibenzo[b,d]furan-4-yl)phenyl)-[1,1':4',1"-terphenyl]-4-amine, CAS 1198399-61-9) was vacuum deposited on the HTL, to form an electron blocking layer (EBL) having a thickness of 5 nm.

Afterwards, 97 volume % of blue emitter host H09 was deposited, and 3 vol.-% of fluorescent blue emitter BD200 (both compounds commercially available from Sun Fine Chemicals, Korea) were deposited on the EBL, to form a blue-emitting EML with a thickness of 20 nm. Then, the electron transporting layer is formed on the hole blocking layer with a thickness of 31 nm by co-deposition of the selected compound according to invention with lithium quinolate (LiQ) in a wt% ratio of 1:1.

Then, on top of the electron transport layer, an electron injection layer is formed by vacuum depositing Yb with a thickness of 2 nm.

Ag is evaporated at a rate of 0.01 to 1 Å/s at 10⁻⁷ mbar and deposited on top of the ytterbium EIL to form a cathode with a thickness of 11 nm.

A cap layer of F1 is formed on the cathode with a thickness of 75 nm.

### Formulae of the auxiliary materials used in device preparation:

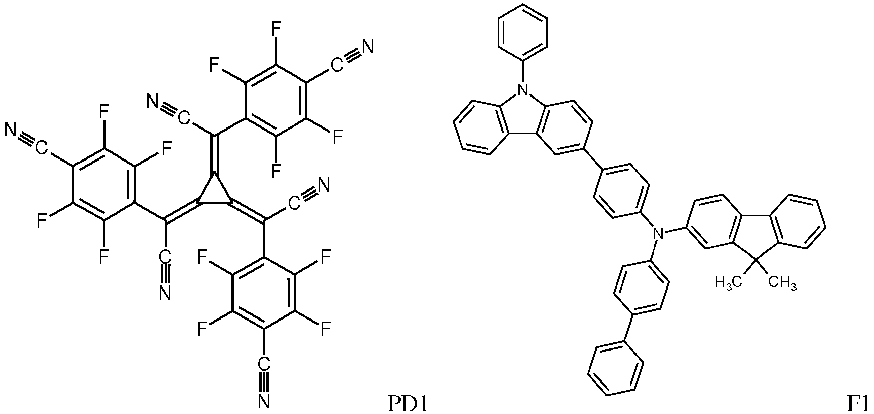

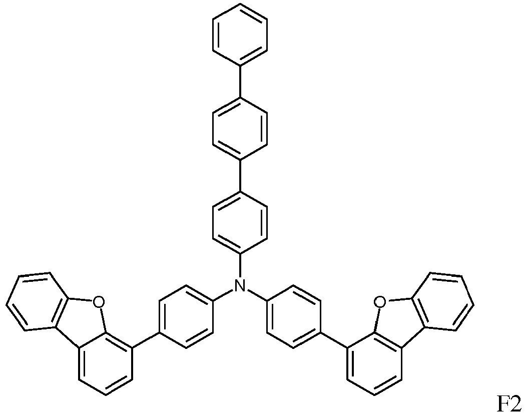

The finished OLED stack is protected from ambient conditions by encapsulation of the device with a glass slide. Thereby, a cavity is formed, which includes a getter material for further protection.

### Means and methods:

### Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

### Device testing procedures

To assess the performance of the inventive examples compared to the existing art, the light output of the top emission OLEDs is measured under ambient conditions (20°C). Current voltage measurements are performed using a Keithley 2400 sourcemeter, and recorded in V at 10 mA/cm² for top emission devices, a spectrometer CAS140 CT from Instrument Systems, which has been calibrated by Deutsche Akkreditierungsstelle (DAkkS), is used for measurement of CIE coordinates and brightness in Candela. The current efficiency Ceff is determined at 10 mA/cm2 in cd/A.

In top emission devices, the emission is forward-directed, non-Lambertian and also highly dependent on the micro-cavity. Therefore, the external quantum efficiency (EQE) and power efficiency in lm/W will be higher compared to bottom emission devices.

### Technical Effect of the invention

In order to investigate the usefulness of the inventive compound six preferred materials were tested in model top-emission blue OLEDs prepared as described above. The results are shown in the Table 1 below:

**Table 1: Properties of several inventive materials and OLEDs comprising such materials**

| compound | Tg [°C] | Voltage [V] | Rel. curr. efficiency [%] | Rel lifetime [%] |
|---|---|---|---|---|
| 1-1 | 147 | 3.76 | 84 | 224 |
| 1-4 | 124 | 3.50 | 97 | 115 |
| 1-3 | 129 | 3.67 | 90 | 158 |
| 1-2 | 136 | 3.49 | 96 | 126 |
| 1-28 | 133 | 4.10 | 89 | 278 |
| 1-47 | 105 | 3.61 | 94 | |

The results show that in comparison with state-of-art reference, the compounds according to invention enable significantly longer device lifetimes at the expense of only very slight efficience decrease.

The particular combinations of elements and features in the above detailed embodiments are exemplary only.

Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims.

Furthermore, reference signs used in the description and claims do not limit the scope of the invention as claimed.

## Claims

1. A compound for an electroluminescent device comprising at least one structural moiety A and at least one structural moiety B, wherein A and B do not share a common atom,
whereby the structural moiety A is a structural moiety having generic formula (I) 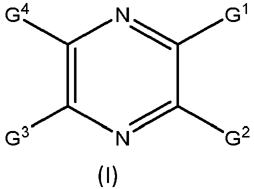
wherein G¹, G², G³ and G⁴ are independently selected from substituted or unsubstituted aryl comprising one to three aromatic rings,
and wherein the structural moiety B is a structural moiety selected from substituted or unsubstituted phosphine oxide, phosphine sulfide, pyrimidine, pyridazine, naphthtalene, cinnoline, phtalazine, quinazoline, triazine, benzofurane, benzothiophene, dibenzofurane, dibenzothiophene, naphtofurane, naphtothiophene, naphtobenzofurane, naphtobenzothiophene, dinaphtofurane, dinaphtothiophene, C₆-C₆₀ aryl substituted with at least one group selected from pyridyl and nitrile, aryl consisting of 3, 4 or 5 condensed 6-membered aromatic rings and heteroaryl consisting of 3, 4 or 5 condensed 6-membered aromatic rings and comprising 1, 2 or 3 nitrogen ring atoms, **characterized in that** one of the G¹, G², G³ and G⁴ is a phenylene unit and the others of the G¹, G², G³ and G⁴ are phenyl.

2. The compound of claim 1, comprising only one structural moiety A.

3. The compound of claim 1 or 2, comprising one or two structural moieties B.

4. The compound of any of the claims 1 to 3 whereby at least one moiety A is connected with at least one moiety B via a single bond or a phenylene, biphenylene or terphenylene bridge.

5. The compound of any of the claims 1 to 4, whereby at least one of the G¹, G², G³ and G⁴ is substituted with at least one fluor, alkyl, fluoroalkyl, nitrile, phosphine sulfide and/ or phosphine oxide group.

6. The compound of any of the claims 1 to 5 whereby at least one moiety B is futher substituted with at least one fluor, alkyl, fluoroalkyl, nitrile, phosphine sulfide and/ or phosphine oxide group.

7. An electronic device (100, 300, 400) comprising a first electrode (150), a second electrode (110), and arranged between the first and second electrode, a layer comprising a compound according to any of the preceding claims 1 to 6.

8. The electronic device of claim 7, whereby the layer comprising a compound according to any of the preceding claims 1 to 6 is a hole blocking layer (33).

9. The electronic device of claim 7 or 8 whereby the electronic device comprises an electron transport layer (31) comprising a compound according to any of the preceding claims 1 to 6.

10. The electronic device according to any of claims 7 to 9, wherein the layer comprising the compound according to any of the preceding claims 1 to 6 further comprises an electrical dopant.

11. The electronic device according to claim 10, wherein the electrical dopant is a n-dopant selected from elemental metals, metal complexes and metal salts.

12. The electronic device according to claim 11,
wherein the n-dopant is selected from salts and/or complexes of alkali metals, alkaline earth metals, and transition metals, preferably from salts and/or complexes of Li, Na, K, Rb, Cs, Mg, Ca, Sr, Ba, Fe, Mn, Zn.

13. The electronic device according to any of the claims 7 to 12, wherein the electronic device is an electroluminescent device, preferably an organic light emitting diode (100).

14. A display device comprising an electronic device according to any of the preceding claims 7 to 13, preferably, the display device comprises an organic light emitting diode (100) according to claim 13.

## Patentansprüche

1. Verbindung für eine Elektrolumineszenzvorrichtung, umfassend mindestens eine Struktureinheit A und mindestens eine Struktureinheit B, wobei sich A und B kein gemeinsames Atom teilen,
wobei die Struktureinheit A eine Struktureinheit der allgemeinen Formel (I) 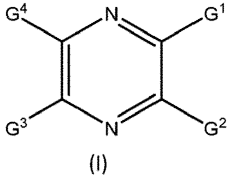
ist, wobei G¹, G², G³ und G⁴ unabhängig aus substituiertem oder unsubstituiertem Aryl umfassend einen bis drei aromatische Ringe ausgewählt sind
und wobei die Struktureinheit B eine Struktureinheit ist, die ausgewählt ist aus substituiertem oder unsubstituiertem Phosphinoxid, Phosphinsulfid, Pyrimidin, Pyridazin, Naphtalin, Cinnolin, Phthalazin, Chinazolin, Triazin, Benzofuran, Benzothiophen, Dibenzofuran, Dibenzothiophen, Naphthofuran, Naphthothiophen, Naphthobenzofuran, Naphthobenzothiophen, Dinaphthofuran, Dinaphthothiophen, C₆-C₆₀-Aryl das durch mindestens eine aus Pyridyl und Nitril ausgewählte Gruppe substituiert ist, Aryl bestehend aus 3, 4 oder 5 kondensierten 6-gliedrigen aromatischen Ringen und Heteroaryl bestehend aus 3, 4 oder 5 kondensierten 6-gliedrigen aromatischen Ringen und aufweisend 1, 2 oder 3 Stickstoff-Ringatome, **dadurch gekennzeichnet, dass** eines von G¹, G², G³ und G⁴ eine Phenyleneinheit ist und die anderen von G¹, G², G³ und G⁴ Phenyl sind.

2. Verbindung nach Anspruch 1, umfassend nur eine Struktureinheit A.

3. Verbindung nach Anspruch 1 oder 2, umfassend eine oder 2 Struktureinheiten B.

4. Verbindung nach einem der Ansprüche 1 bis 3, wobei mindestens eine Einheit A über eine Einfachbindung oder eine Phenylen-, Biphenylen- oder Terphenylenbrücke mit mindestens einer Einheit B verbunden ist.

5. Verbindung nach einem der Ansprüche 1 bis 4, wobei mindestens eines von G¹, G², G³ und G⁴ durch mindestens eine Fluor-, Alkyl-, Fluoralkyl-, Nitril-, Phosphinsulfid- und/oder Phosphinoxidgruppe substituiert ist.

6. Verbindung nach einem der Ansprüche 1 bis 5, wobei mindestens eine Einheit B weiter durch mindestens eine Fluor-, Alkyl-, Fluoralkyl-, Nitril-, Phosphinsulfid- und/oder Phosphinoxidgruppe substituiert ist.

7. Elektronische Vorrichtung (100, 300, 400) mit einer ersten Elektrode (150), einer zweiten Elektrode (110) und einer zwischen der ersten und zweiten Elektrode angeordneten Schicht, die eine Verbindung nach einem der vorhergehenden Ansprüche 1 bis 6 umfasst.

8. Elektronische Vorrichtung nach Anspruch 7, wobei die Schicht, die eine Verbindung nach einem der vorhergehenden Ansprüche 1 bis 6 umfasst, eine Lochblockierschicht (33) ist.

9. Elektronische Vorrichtung nach Anspruch 7 oder 8, wobei die elektronische Vorrichtung eine Elektronentransportschicht (31) umfasst, welche eine Verbindung nach einem der Ansprüche 1 bis 6 umfasst.

10. Elektronische Vorrichtung nach einem der Ansprüche 7 bis 9, wobei die Schicht, die die Verbindung nach einem der vorhergehenden Ansprüche 1 bis 6 umfasst, ferner einen elektrischen Dotanden umfasst.

11. Elektronische Vorrichtung nach Anspruch 10, wobei der elektronische Dotand ein n-Dotande ist, der aus elementaren Metallen, Metallkomplexen und Metallsalzen ausgewählt ist.

12. Elektronische Vorrichtung nach Anspruch 11, wobei der n-Dotand aus Salzen und/oder Komplexen von Alkalimetallen, Erdalkalimetallen und Übergangsmetallen, vorzugsweise aus Salzen und/oder Komplexen von Li, Na, K, Rb, Cs, Mg, Ca, Sr, Ba, Fe, Mn, Zn, ausgewählt ist.

13. Elektronische Vorrichtung nach einem der Ansprüche 7 bis 12, wobei die elektronische Vorrichtung eine Elektrolumineszenzvorrichtung ist, vorzugsweise eine organische Leuchtdiode (100).

14. Anzeigevorrichtung, umfassend eine elektronische Vorrichtung nach einem der vorhergehenden Ansprüche 7 bis 13, wobei die Anzeigevorrichtung vorzugsweise eine organische Leuchtdiode (100) nach Anspruch 13 umfasst.

## Revendications

1. Composé pour un dispositif électroluminescent comprenant au moins un groupement structural A et au moins un groupement structural B, A et B ne partageant pas un atome commun, dans lequel le groupement structural A est un groupement structural ayant une formule générale (I) 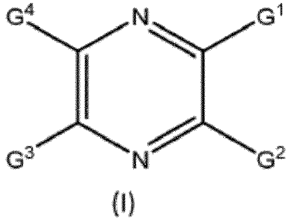
G¹, G², G³ et G⁴ étant indépendamment choisis parmi aryle substitué ou non substitué comprenant un à trois cycles aromatiques,
et le groupement structural B étant un groupement structural choisi parmi oxyde de phosphine, sulfure de phosphine, pyrimidine, pyridazine, naphtalène, cinnoline, phtalazine, quinazoline, triazine, benzofurane, benzothiophène, dibenzofurane, dibenzothiophène, naphtofurane, naphtothiophène, naphtobenzofurane, naphtobenzothiophène, dinaphtofurane, dinaphtothiophène substitué(e) ou non substitué (e), C₆₋₆₀ aryle substitué par au moins un groupe choisi parmi pyridinyle et nitrile, aryle constitué de 3, 4 ou 5 cycles aromatiques à 6 chaînons condensés et hétéroaryle constitué de 3, 4 ou 5 cycles aromatiques à 6 chaînons condensés et comprenant 1, 2 ou 3 atomes de cycle de type azote, **caractérisé en ce que** l'un parmi les G¹, G², G³ et G⁴ est un motif phénylène et les autres parmi les G¹, G², G³ et G⁴ sont phényle.

2. Composé selon la revendication 1, comprenant seulement un groupement structural A.

3. Composé selon la revendication 1 ou 2, comprenant un ou deux groupements structuraux B.

4. Composé selon l'une quelconque des revendications 1 à 3, dans lequel au moins un groupement A étant relié à au moins un groupement B via une liaison simple ou un pont phénylène, biphénylène ou terphénylène.

5. Composé selon l'une quelconque des revendications 1 à 4, dans lequel au moins l'un parmi les G¹, G², G³ et G⁴ étant substitué par au moins un groupe fluor, alkyle, fluoroalkyle, nitrile, sulfure de phosphine et/ou oxyde de phosphine.

6. Composé selon l'une quelconque des revendications 1 à 5, dans lequel au moins un groupement B étant en outre substitué par au moins un groupe fluor, alkyle, fluoroalkyle, nitrile, sulfure de phosphine et/ou oxyde de phosphine.

7. Dispositif électronique (100, 300, 400) comprenant une première électrode (150), une deuxième électrode (110), et agencée entre la première et la deuxième électrode, une couche comprenant un composé selon l'une quelconque des revendications précédentes 1 à 6.

8. Dispositif électronique selon la revendication 7, dans lequel la couche comprenant un composé selon l'une quelconque des revendications précédentes 1 à 6 étant une couche de blocage de trous (33).

9. Dispositif électronique selon la revendication 7 ou 8, dans lequel le dispositif électronique comprenant une couche de transport d'électrons (31) comprenant un composé selon l'une quelconque des revendications précédentes 1 à 6.

10. Dispositif électronique selon l'une quelconque des revendications 7 à 9, dans lequel la couche comprenant le composé selon l'une quelconque des revendications précédentes 1 à 6 comprenant en outre un dopant électrique.

11. Dispositif électronique selon la revendication 10, dans lequel le dopant électrique étant un dopant n choisi parmi des métaux élémentaires, des complexes métalliques et des sels métalliques.

12. Dispositif électronique selon la revendication 11, dans lequel le dopant n étant choisi parmi des sels et/ou des complexes de métaux alcalins, de métaux alcalino-terreux et de métaux de transition, préférablement des sels et/ou des complexes de Li, Na, K, Rb, Cs, Mg, Ca, Sr, Ba, Fe, Mn, Zn.

13. Dispositif électronique selon l'une quelconque des revendications 7 à 12, dans lequel le dispositif électronique étant un dispositif électroluminescent, préférant une diode émettrice de lumière organique (100).

14. Dispositif d'affichage comprenant un dispositif électronique selon l'une quelconque des revendications précédentes 7 à 13, préférablement, le dispositif d'affichage comprenant une diode émettrice de lumière organique (100) selon la revendication 13.
